Europäisches Patentamt

European Patent Office

Office européen des brevets

(11) Publication number: **0 501 411 A1**

## EUROPEAN PATENT APPLICATION

(21) Application number: **92103159.7**

(22) Date of filing: **25.02.92**

(51) Int. Cl.⁵: **A47C 9/00**

(30) Priority: **26.02.91 IL 97364**

(43) Date of publication of application:
**02.09.92 Bulletin 92/36**

(84) Designated Contracting States:
**AT BE CH DE DK ES FR GB IT LI NL PT SE**

(71) Applicant: **Ramon, Arie**
**Mishol Hahadas 11, Ramot 2**
**Jerusalem(IL)**
Applicant: **Almagor, Elath, Dr.**
**Ramot 37**
**Jerusalem(IL)**

(72) Inventor: **Ramon, Arie**
**Mishol Hahadas 11, Ramot 2**
**Jerusalem(IL)**
Inventor: **Almagor, Elath, Dr.**
**Ramot 37**
**Jerusalem(IL)**

(74) Representative: **Kraus, Walter, Dr. et al**
**Patentanwälte Kraus, Weisert & Partner**
**Thomas-Wimmer-Ring 15**
**W-8000 München 22(DE)**

(54) **Seating means.**

(57) There is proposed a body supporting or seating structure which is anatomically beneficial to the human body and also ensures freedom of movement of the person sitting or reclining on the new support. The support comprises a seat (10) which has one single leg which rises from a leg pad. Two rollers or castors are connected to a structure (2) supporting the said single leg.

FIG. 5

EP 0 501 411 A1

## BACKGROUND AND FIELD OF INVENTION

The present invention concerns a support for a person who assumes a sitting or quasi sitting position.

There arc already known devices to support the body of a person who attends to certain functions or occupations which while permitting a certain, more or less free movement obligates the respective person to assume body positions of one kind or another during certain periods of time.

## SHORT DESCRIPTION OF PRIOR ART

Known supports mainly comprise a single supporting leg. As examples may be mentioned U.S. Patent No. 1,417,250 to Kelly which describes a supporting device for workers who most of the time, when working, have to assume a bending or stooping posture. The device consists mainly of a breast plate to be strapped to the body of the worker by shoulder straps and the breast plate being carried on the said single leg. A person using the device may bend over and would be supported by the device on whose breast plate her or his breast region rests while the single leg stands on the ground.

U.S. Patent No. 3,306,658 describes a device for the use of dentists or dental surgeons who - as is known - work on a person who reclines in a special chair. The dentist is supported on a saddle like seat which rests on the top of a single leg. The device allows almost unlimited movement to the user who is also supported on her or his own legs, since the person's feet remains on the ground beside the chair in which the patient reclines.

U.S. Patent No.4,451,080 describes a device for use by paraplegics or those who lack legs. The person sits on and is secured to a seat resting on a single leg and "walks" with the aid of crutches. To permit lateral movement, the single leg has at its lower end a pad whose downward facing surface is curved.

## SHORT STATEMENT OF ADVANTAGES OF INVENTION

In comparison with other above mentioned devices the present invention is intended for much wider use.

Maximum freed of of movement and agility is generally associated with the standing posture which however has the disadvantage of being tiring to those working. The sitting position gives more comfort but severely limits the freedom of movement.

The present invention relates to a seating means which permits the user a greater freedom of movement to a degree which allows more convenient attendance to work which conventionally is performed by normal persons in a sitting position.

The suggested means permit a person to assume a posture closer to standing up as far as freedom of movement is concerned, and yet supports major part of the body weight by the buttocks.

The new seating means according to experimental tests and opinion of anatomical experts is preferable from the points of assuming a healthful, natural posture and enabling frequent small changes in sitting position to the conventional sitting position where the rump region occupies a seat and the upper part of the body is supported by a back rest against which the sitter leans with very little movement.

## SUMMARY OF THE INVENTION

According to the invention the new seating means comprises a seat part which is supported on a single leg having a leg pad with convex, preferably spherical underneath face, to the lower part of said single leg a supporting structure is connected provided with two rollers or castors at the rear end of said structure.

In a practical embodiment of the invention, the length of the single leg and consequently the level at which the seat is held is controllable by making the leg of two into one another telescoping parts.

In comparison with other known one leg support seating devices the new invention has a distinctly different approach to the relation between the centre of curvature of the pad of the leg and the location of the centre of gravity of the sitting person.

The known devices adopt one of the two approaches;

a) small radius of curvature and centre of gravity of the seater much higher than the centre of curvature

b) large radius of curvature and the centre of gravity of the seater below the centre of curvature to create a torque which tends to bring the chair back to certain neutral position and preventing the seater to exceed comfortable and safe angles of incline of the chair.

Experience has shown that the preferred radius of curvature is within 25 - 70 cm.

The present invention intends, within limits of practical constrains, to have the centre of gravity of seater (the seater and chair combined to be precise) to coincide with the centre of curvature of the leg pad (at least in part of the range of movement).

This configuration has the advantage of free movement with minimum strain in the back muscle and stress on the spine.

The disadvantage is that a separate mechanism to prevent exceeding safe angles of chair incline is required.

Limiting legs provide such safety.

These and further features of the invention will become clear from the following detailed description which refers to the accompanying drawings.

## SHORT DESCRIPTION OF DRAWINGS

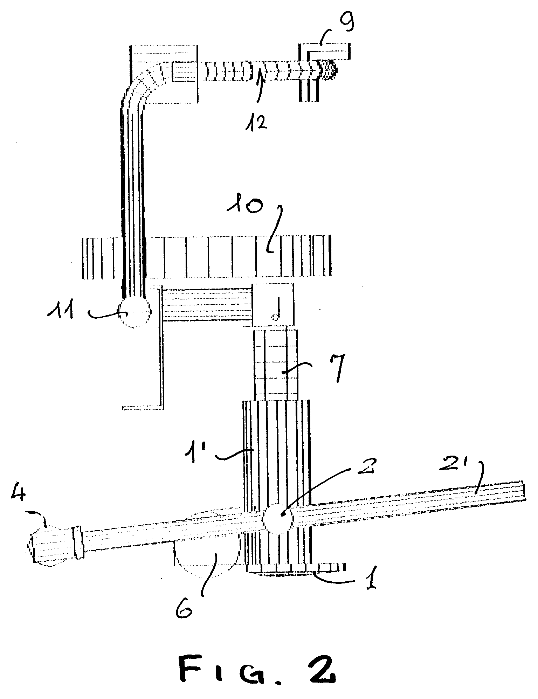

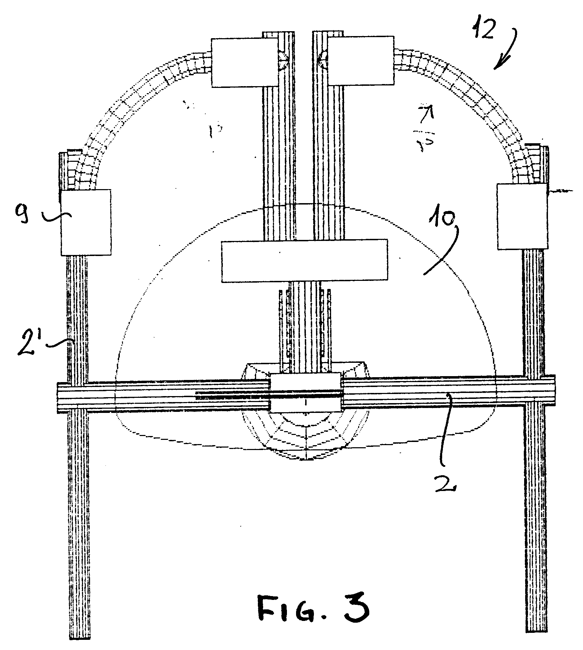

In the drawings the new means is shown in Fig. 1 in a frontal, elevational view and in Fig. 2 in a like lateral view. Fig. 3, finally is a top view of the new seating means. Fig. 4 shows an alternative abdomen supporting means. Fig. 5 is a further embodiment of the supporting means.

## DETAILED DESCRIPTION OF PREFERRED EMBODIMENT

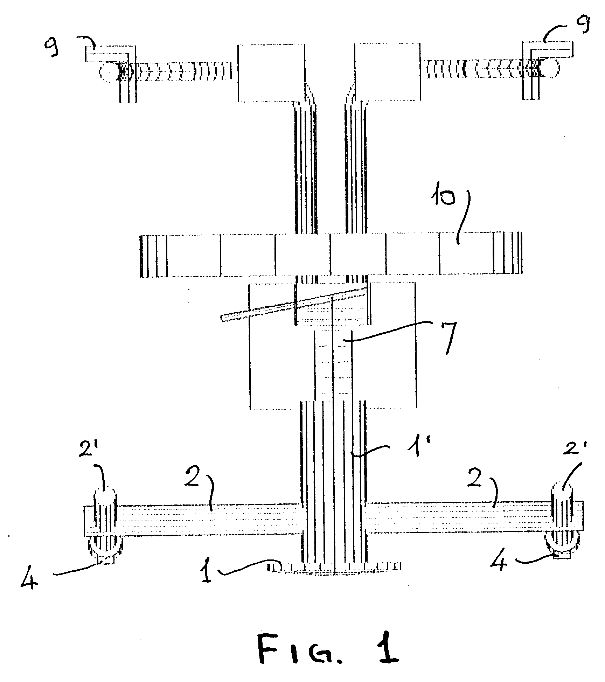

Turning first to Fig. 1, a two part single leg 1' (the two parts telescoping into one another) extends from a foot pad 1. This latter has a convexly curved underside. Horizontally from foot leg part 1' extends to both sides a beam 2 to which is connected another beam 2' at right angles extending forwardly and rearwardly of the seat. To the rear end of beam 2' is affixed a roller or castor 4. A larger wheel 6 is affixed to foot beam 2' near pad 1. The upper part 7 of the leg (which is slidable in the lower base part 1' thereof) carries a seat 10. Slightly below the seat 10, affixed to leg part 7, extends a cross beam 11 which carries arm rests 9.

A person can occupy the seat 10 facing in the direction of an abdomen support 12 consisting of two halves of a body belt. When the two halves of the belt are buckled or connected together the person on seat 10 leans on his or her belly portion onto the closed belt. When sitting on seat 10 with chair upright the person's feet just touch the ground without loading the weight of the body onto that person's legs. When the chair is inclined increasing but still minor part of the weight is transferred to the legs.

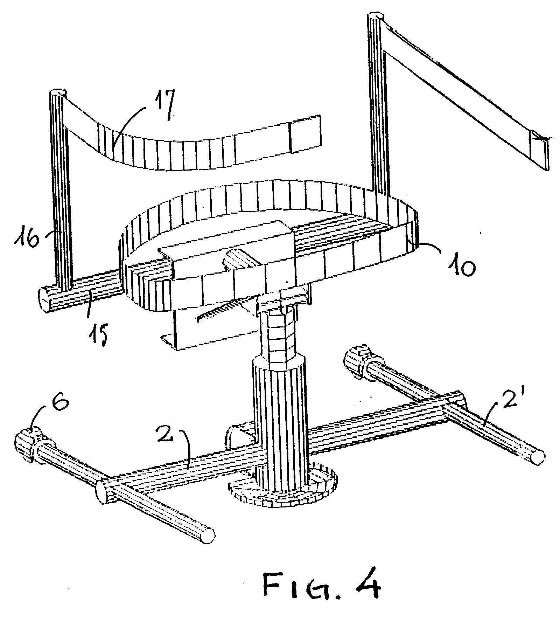

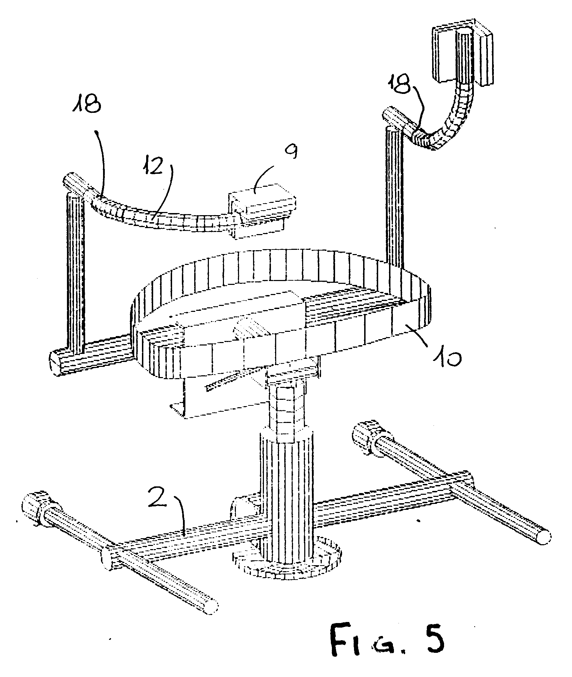

Another, alternative form of abdomen support is shown in Figs. 4 and 5. In this arrangement there extend below seat 10 substantially across the middle thereof, two arms 15 which carry each an upright 16. These latter carry each a bow shaped member 17 which extends in a horizontal plane above seat 10. Arms 15 and with them uprights 16 and the bows 17 are turnable about pivots 18, such that the bows 17 are either at front or rear of the seat and a person can lean against the bows 17 either with her or his abdomen or back.

Optionally the arms 15 can be meshed through gears so that symmetrical motion is caused by movement of one of the arms.

"Overtilting" is prevented by beam 2 either by castors 4 or the skived end of the beam, possibly assisted by stops at the centre of the beam. When the single leg is strictly vertical the two castors 4 are off the ground and the whole chair is supported on the pad 1 and the larger wheel 6. Whenever the person on the seat 10 leans in the rearward direction the front end of beam 2 rises with pad 1 which is no longer in contact with the floor and castors 4 come down to the ground.

It will be seen that the person occupying the chair is safely sitting and can freely move his or her backside within rough oval of more than 10 by 10 cm and rotate the body with no limitation at all. At the same time his or her arms and hands being entirely free to do whatever work. The posture assumed in the chair is natural and might relieve back aches.

Fig. 5 illustrates a further embodiment of the support. When the two halves are in the frontal position, it is possible to swivel the bows at 18 to enable the rising or seating of a person

## Claims

1. A new seating means comprising a seat part which is supported on a single leg having a leg pad with convex, preferably toroidal underneath face, to the lower part of said single leg a supporting structure being connected provided with two rollers or castors at the rear end of said structure and a single wheel at the centre to stabilize the seating means and prevent overturning thereof.

2. A seating means according to claim 1, characterised by said single leg being telescopic.

3. A seating means according to claim 1, characterised by a supporting arrangement being provided to support the abdomen region of a person occupying the seat.

4. A seating means according to claim 3, where said supporting arrangement comprises two bows carried on a structure which is pivotally connected to the seating means such that the bows are either at the front, side, or rear of the seat.

5. A seating means according to claim 3, where the supporting arrangement consists of two halves of a body belt.

6. A seating means according to claim 3, where the supporting arrangement comprises two bows carried on a structure which is pivotally connected to the seating means such that the bows are either in abdomen supporting position or lifted on a horizontal pivot to allow seating or rising.

7. A seating means according to claim 1, characterised thereby that the radius of curvature of said paid is from 25 - 70 cm.

FIG. 1

FIG. 2

FIG. 3

FIG. 4

FIG. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-C-48 348 (NEUERBURG) <br> * the whole document * | 1,2,7 | A47C9/00 |
| A | DE-C-825 144 (FESCA) <br> * the whole document * | 1,2,7 | |
| A | DE-A-3 207 941 (RAU) <br> * page 5, line 16 - page 7, line 7; figures 1,4 * | 1,2 | |
| A | FR-A-1 231 955 (LESIEUR) <br> * the whole document * | 1,2 | |
| A | FR-A-1 480 037 (FABRE) <br> * page 1, right column, paragraph 8 - page 2, right column, paragraph 3; figures * | 1,2 | |
| A | US-A-3 754 787 (GARBER) <br> * figures * | 1,3 | |
| | ----- | | **TECHNICAL FIELDS SEARCHED (Int. Cl.5)** <br><br> A47C |

The present search report has been drawn up for all claims

| Place of search | Date of completion of the search | Examiner |
|---|---|---|
| THE HAGUE | 23 APRIL 1992 | DE COENE P.J.S. |

EPO FORM 1503 03.82 (P0401)